# EUROPEAN PATENT SPECIFICATION

(11) **EP 4 142 592 B1**
(45) Date of publication and mention of the grant of the patent: **26.03.2025**
(21) Application number: 21797813.9
(22) Date of filing: 26.04.2021
(51) Int. Cl.: A61B 5/24, H03G 3/20, A61B 5/30, A61B 5/00, A61B 5/0531

(54) **APPARATUS AND METHOD FOR DETECTION OF BIOPOTENTIAL SIGNALS**
VORRICHTUNG UND VERFAHREN ZUR ERKENNUNG VON BIOPOTENZIALSIGNALEN
APPAREIL ET PROCÉDÉ DE DÉTECTION DE SIGNAUX DE BIOPOTENTIEL

(30) Priority: 27.04.2020 DK PA202070266
(43) Date of publication of application: 08.03.2023
(73) Proprietor: Paragit Solutions ApS, 1864 Frederiksberg C (DK)
(72) Inventor: FILFIL, Mohammad, 2650 Hvidovre (DK)
(74) Representative: Zacco Denmark A/S
(86) International application number: PCT/DK2021/050129
(87) International publication number: WO 2021/219183

(56) References cited:
- JP-A- 2018 094 412
- US-A1- 2002 109 621
- US-A1- 2011 237 904
- US-A1- 2014 142 447
- US-A1- 2015 327 815
- US-A1- 2019 008 383

## Description

### TECHNICAL FIELD

The disclosure relates to an electronic medical device and method, more particularly to an apparatus and method for accurately and continuously measuring a bio-signal, e.g. biopotential signal.

### BACKGROUND

The detection of bio-signals such as e.g. biopotential has long been used in medical and therapeutic environments to detect, diagnose and monitor the progression of diseases. The best known and most commonly detected bio-signals are Electroencephalogram (EEG), Electrocardiogram (ECG), and Electromyogram (EMG) bio-signals. Recent technological advancements in electronics, signal processing, artificial intelligence, wireless technologies, and smart wearables created numerous opportunities for integration of bio-signal measuring and processing, enabling the user to access more everyday-friendly apparatuses for monitoring their condition or disease.

Although several approaches are known for bio-signal monitoring, the monitoring of bio-signals detected on the surface of the skin has been problematic due impedance changes caused by external factors such as motion or movement artifacts and dust, dirt, oil, and sweat among others. The effect of these factors is significant enough that it may create an inability to monitor the required electrical event. Many efforts have been made to overcome the issues arising from impedance changes caused by external factors.

US20170312576 discloses a wearable system and method for analyzing physical activity of a user for training and/or therapeutic purpose by analyzing multiple channels of data from non-invasive surface electromyography (sEMG) and inertial measurement units (IMU).

US20080275316 discloses a switch-network system for matching skin impedance and a method for skin/electrode interface. Further, US 2011/0251817 discloses a method and apparatus to determine impedance variations in a skin/electrode interface. US20110060252 describes an apparatus and method for monitoring persons suffering from epilepsy, Parkinson's disease, or the like disease as well as other conditions in active (e.g. during travelling in a vehicle) and passive (e.g. hospital) settings.

EP2294979 describes a method and device for measuring biopotential signals together with an impedance signal on a subject's skin with reduced power consumption. US20110237904 describes a method and device for sensing a biopotential signal on the skin of a person with a variable controlled gain.

None of these known apparatuses are able to compensate for both impedance changes caused both by movement artifacts and by sweat accumulation on the skin nor can they deviate and identify which exact electrodes are displaced or shunted.

### SUMMARY

It is an object to provide a method and apparatus for measuring a biopotential signal that overcomes or at least reduces the problems mentioned above.

The foregoing and other objects are achieved by the features of the independent claims. Further implementation forms are apparent from the dependent claims, the description, and the figures.

According to a first aspect, there is provided an apparatus for sensing a biopotential signal on the skin of a person using at least a first skin electrode, a second skin electrode and a reference skin electrode for spaced placement on the skin of the person, the apparatus comprising: a first terminal for connection to the first electrode, a second terminal for connection to the second electrode, a first circuitry configured for measuring the biopotential signal from the first and the second terminal, a third terminal for connection to the reference skin electrode, a first variable controlled resistance load connected to the first terminal, a second variable controlled resistance load connected to the second terminal, characterized by, a first signal generator for generating a first alternating signal connected to the first terminal, a second signal generator for generating a second alternating signal connected to the second terminal, a fourth circuitry connected to the output of the first circuitry and configured to apply a variable controlled gain to the output signal of the first circuitry, a controller in receipt of: an output signal of the first circuitry, a signal comprising a component representative of the amplitude of the first alternating signal, a signal comprising a component representative of the amplitude of the second alternating signal, the controller being configured: to determine the amplitude of the first alternating signal, to determine the amplitude of the second alternating signal, to control the first variable controlled resistance load and the second variable controlled resistance load, to control the variable controlled gain, to adapt the load of the first variable controlled resistance load as a function of a change in the determined amplitude of the first alternating signal, to adapt the load of the second variable controlled resistance load as a function of a change in the determined amplitude of the second alternating signal, and to adjust the variable controlled gain as a function of the determined amplitude of the first alternating signal and of the determined amplitude of the second alternating signal, when, and only when, the determined amplitude of both the first alternating signal and the second alternating signal change simultaneously.

By providing a first variable controlled resistance load associated with the first signal and by providing a second variable controlled resistance load associated with the second signal, and by adjusting the respective variable controlled resistance load in response to a change in amplitude of the respective signal, it becomes possible to fully compensate for movement artifacts, and by adjusting the variable controlled gain in response to a simultaneous change in amplitude of the output signal of both the second and third circuitry, it becomes possible to fully compensate for the shunting effect of sweat on the skin of the user.

In a possible implementation form of the first aspect, the signal comprising at least a component representative of the amplitude of the first alternating signal, and the signal comprising a component representative of the amplitude of the second alternating signal is the output signal of the first circuitry.

By providing two known, but different alternating signals it becomes possible to differentiate between the amplitude of the first alternating signal and the amplitude of the second alternating signal, allowing for the individual control of the first and second variable resistance loads.

In a possible implementation form of the first aspect, the controller being configured: to perform a time-frequency analysis of the output signal of the first circuitry and thereby determine the portion of the output signal of the first circuitry originating from the first alternating signal and the portion of the output signal of the first circuitry originating from the second alternating signal, to determine the amplitude of the output signal of the first circuitry originating from the first alternating signal, to determine the amplitude of the output signal of the first circuitry originating from the second alternating signal, to control the first variable controlled resistance load, to control the second variable controlled resistance load, to control the variable controlled gain, to adapt the load of the first variable controlled resistance load as a function of the amplitude of the output signal of the first circuitry originating from the first alternating signal, to adapt the load of the second variable controlled resistance load as a function of the amplitude of the output signal of the first circuitry originating from the second alternating signal, and to adjust the variable controlled gain as a function of the amplitude of the output signal of the first circuitry originating from the first alternating signal and of the amplitude of the output signal of the first circuitry originating from the second alternating signal when, and only when, the amplitude of both the output signal of the first circuitry originating from the first alternating signal and the amplitude of the output signal of the first circuitry originating from the second alternating signal change simultaneously.

By having a controller capable of performing calculations in the time-frequency domain (e.g. to perform a fast Fourier transformation) it becomes possible to analyze and differentiate between the origins of the output signal of the first circuitry for controlling the variable resistance loads individually and independently of each other as a function of the result of the calculations.

In a possible implementation form of the first aspect, the apparatus further comprising a second circuitry connected to the first terminal for sensing and/or amplifying the amplitude of the first signal and a third circuitry connected to the second terminal for sensing and/or amplifying the amplitude of the second signal, a controller in receipt of the output signal of the second circuitry and in receipt of the output signal of the third circuitry, the controller being configured: to adapt the load of the first variable controlled resistance load as a function of the amplitude of the output signal of the second circuitry, to adapt the load of the second variable controlled resistance load as a function of the amplitude of the output signal of the third circuitry, and to adjust the variable controlled gain as a function of the amplitude of the output signal of the second circuitry and of the amplitude of the output signal of the third circuitry when, and only when, the amplitude of both the output signal of both the second circuitry and the third circuitry change simultaneously.

By providing a first variable controlled resistance load associated with the first signal and by providing a second variable controlled resistance load associated with the second signal, and by adjusting the respective variable controlled resistance load in response to a change in amplitude of the respective signal, it becomes possible to fully compensate for movement artifacts, and by adjusting the variable controlled gain in response to a simultaneous change in amplitude of the output signal of both the second and third circuitry, it becomes possible to fully compensate for the shunting effect of sweat on the skin of the user.

In a possible implementation form of the first aspect, the controller is configured to increase the variable controlled gain when and only when the amplitude of both the output signal of the second and the third circuitry attenuate simultaneously.

In a possible implementation form of the first aspect, the controller is configured to decrease the variable controlled gain when and only when the amplitude of both the output signal of the second and third circuitry increase simultaneously.

In a possible implementation form of the first aspect, the controller is configured to set the first variable controlled resistance load and the second variable controlled resistance load both to a medium value, and that the controller is configured to thereupon calibrate the apparatus as a function of the amplitude of the output signal of the second circuitry and as a function of the output signal of the third circuitry, and wherein the controller preferably is configured to store a first initial calibration value associated with the first alternating signal and preferably is configured to store a second initial calibration value associated with the second alternating signal.

By setting the first and second variable resistance load to a medium value and storing the initial calibration value associated with the first and second alternating signals, it becomes possible to easily and quickly determine any change in the amplitude of the output signal of the second and/or the third circuitry and to compensate for such changes.

In a possible implementation form of the first aspect, the controller is configured to update the first and second initial calibration value after the controller has adjusted the variable controlled gain.

In a possible implementation form of the first aspect, the first circuity comprises an instrumental amplifier connected to the first and second terminal for amplifying the biopotential signal.

In a possible implementation form of the first aspect, the second circuitry comprises a first amplifier connected to the first terminal for amplifying the amplitude of the first signal, the first amplifier preferably being an operational amplifier.

In a possible implementation form of the first aspect, the third circuitry comprises a second amplifier connected to the second terminal for amplifying the amplitude of the second signal, the second amplifier preferably being an operational amplifier.

By providing a first and a second amplifier it becomes possible to individually measure and compensate for signal attenuation of the first and second electrodes.

In a possible implementation form of the first aspect, the fourth circuitry comprises a digitally controlled amplifier connected to the output of the instrumental amplifier and configured to apply a variable controlled gain to the output signal of the instrumental amplifier.

In a possible implementation form of the first aspect, the controller is in receipt of the output signal of the first amplifier and in receipt of the output of the second amplifier, the controller being configured to increase the resistance of the first variable controlled resistance load when the amplitude of the output of the first amplifier decreases and configured to decrease the resistance of the first variable controlled resistance load when the amplitude of the output of the first amplifier increases, and the controller being configured to increase the resistance of the second variable controlled resistance load when the amplitude of the output of the second amplifier decreases and configured to decrease the resistance of the second variable controlled resistance load when the amplitude of the output of the second amplifier increases.

In a possible implementation form of the first aspect, the first, the second and/or the reference electrodes are dry surface electrodes.

In a possible implementation form of the first aspect, the first signal has a first frequency, the second signal has a second frequency, the first frequency preferably being equal to the second frequency, and the first signal preferably being out of phase with the second signal.

In a possible implementation form of the first aspect, the controller is configured to adjust the variable controlled gain as a function of the amplitude of the output signal of the second circuitry and/or of the amplitude of the output signal of the third circuitry when, and only when, the amplitude of both the output signal of both the second circuitry and the third circuitry change simultaneously and in the same direction.

In a possible implementation form of the first aspect, the controller is configured to calibrate the apparatus by measuring and recording the output of the second circuitry, the third circuitry and the fourth circuitry, preferably in relation to a default setting of the first aspect.

In a possible implementation form of the first aspect, the controller is configured to set the first variable controlled resistance load and the second variable controlled resistance load both to a medium value, and that the controller is configured to thereupon calibrate the apparatus as a function of the amplitude of the output signal of the second circuitry and as a function of the output signal of the third circuitry.

By calibrating the apparatus during a first, initial use it is possible to record accurate measurements of a person's bio-signals over time while continuously compensating for environmental effects affecting the signal strength and quality. Calibration of the apparatus further provides an advantage of providing an individual measurement and reference value for a bio-signal of a person and allows for comparison and detection of any deviation from this calibrated value during a bio-signal measurement of a person.

In a possible implementation form of the first aspect, the controller is configured to set the first variable controlled resistance load and the second variable controlled resistance load both to a medium value, and the controller is configured to thereupon calibrate the apparatus as a function of the amplitude of the output signal of the first and second terminal.

In a possible implementation form of the first aspect, the controller may be a microprocessor capable of achieving high effective performance.

By employing a powerful yet economical microprocessor it becomes possible to eliminate some components as the computing power and performance is sufficient for carrying out the necessary measurements and calculations without the need for additional e.g. amplifiers. Suitable microprocessors may be e.g. ARM Cortex-M processors. The microprocessor and its performance may also determine the need and configuration of amplifiers used in the apparatus.

In a possible implementation form of the first aspect, the controller is configured to operate in the frequency domain.

In a possible implementation form of the first aspect the controller is configured to adapt the load of the second variable controlled resistance load as a function of the amplitude of the output signal of the third circuitry, and to adjust the variable controlled gain as a function of the amplitude of the output signal of the second circuitry and/or of the amplitude of the output signal of the third circuitry when, and only when, the amplitude of both the output signal of the second circuitry and the third circuitry change simultaneously, and in the same direction.

In a possible implementation form of the first aspect, the second signal generated by the second signal generator is out of phase with the first signal generated by the first signal generator, preferably between 40 and 320° out of phase, more preferably approximately 120° out of phase.

By providing an individual signal for the first electrode and an individual signal for the second electrode and by monitoring the signals individually, as well as relative to one another, it becomes possible to determine whether changes in impedance are caused by movement artifacts affecting the first electrode or whether changes in impedance are caused by movement artifacts affecting the second electrode or weather changes in impedance are caused by movement artifacts affecting both the first and second electrode, or whether changes in impedance are caused by a simultaneous, same rate decrease of each individual signal due to the shunting effect of sweat accumulation. With this insight, it becomes possible to provide an apparatus that can accurately measure three varying resistances ΔR₁, ΔR₂ and R₃ and to effectively compensate impedance changes caused by movement artifacts and sweat accumulation, by updating the variable controlled gain and impedance balance as required. ΔR₁ and ΔR₂ is understood to be the variable resistance measured between the first electrode and second electrode, respectively, and the skin due to movement artifacts and electrode displacement. R₃ is understood to be the decreased surface skin resistance between the first and second electrodes as a result of accumulation of sweat.

In a possible implementation form of the first aspect, the controller is configured to increase the variable controlled gain when and only when the amplitude of both the output signal of the second and third circuitry attenuate simultaneously.

In a possible implementation form of the first aspect, the controller is configured to decrease the variable controlled gain when and only when the amplitude of both the output signal of the second and third circuitry increases simultaneously.

In a possible implementation form of the first aspect, the controller is configured to set the first variable controlled resistance load and the second variable controlled resistant load both to a medium value, and that the controller is configured to thereupon calibrate the apparatus as a function of the amplitude of the output signal of the second circuitry and as a function of the output signal of the third circuitry, and wherein the controller is preferably configured to store a first initial calibration value associated with the first alternating signal and wherein the controller is preferably configured to store a second initial calibration value associated with the second alternating signal.

In a possible implementation form of the first aspect, the controller is configured to update the first and second initial calibration value after the controller has adjusted the variable controlled gain.

In a possible implementation form of the first aspect, the first circuitry comprises an instrumental amplifier connected to the first and second terminal for amplifying the biopotential signal.

In a possible implementation form of the first aspect, the second circuitry comprises a first amplifier connected to the first terminal for amplifying the amplitude of the first signal, the first amplifier preferably being an operational amplifier.

In a possible implementation form of the first aspect, the third circuitry comprises a second amplifier connected to the second electrode for amplifying the amplitude of the second signal, the second amplifier preferably being an operational amplifier.

In a possible implementation form of the first aspect the fourth circuitry comprises a digitally controlled amplifier connected to the output of the instrumental amplifier and configured to apply a variable controlled gain to the output signal of the instrumental amplifier.

In a possible implementation form of the first aspect, the controller is configured to receive the signal outputs from the first amplifier to control the first variable controlled resistance load and is configured to receive the signal outputs from the second amplifier to control the second variable controlled resistance load and further, the controller is configured to control the third amplifier either by hardware means or by using a software.

According to a second aspect, there is provided a method for sensing a biopotential signal on the skin of a person, the method comprising: placing a first skin electrode, a second skin electrode and a reference skin electrode on the skin of the person, applying a first alternating signal to the first electrode, connecting a first variable controlled resistance load to the first electrode, connecting a second variable controlled resistance load to the second electrode, characterized by, measuring the amplitude of the first signal and adjusting the resistance of the first variable controlled resistance load, as a function of a change in amplitude of the first signal, applying a second alternating signal to the second electrode, measuring the amplitude of the second signal and adjusting the resistance of the second variable controlled resistance load as a function of a change in amplitude of the second signal, measuring the biopotential signal with the first and second electrode and applying a variable controlled gain to the measured biopotential signal, and adjusting the variable controlled gain as a function of a change in amplitude of the measured first signal and of the amplitude of the measured second signal when and only when the amplitude of both the first signal and the amplitude of the second signal changes simultaneously.

In a possible implementation form of the second aspect, measuring the amplitude of the first signal and adjusting the resistance of the first variable controlled resistance load when the amplitude of the first signal attenuates to match the impedance from the first circuitry.

In a possible implementation form of the second aspect, measuring the amplitude of the second signal and adjusting the resistance of the second variable controlled resistance load when the amplitude of the second signal attenuate to match impedance from the first circuitry.

In a possible implementation form of the second aspect, setting the first variable controlled resistance load and the second variable controlled resistance load to approximately half the maximum of the fully operational range of the respective variable controller resistance load and measuring the amplitude of the first signal and a measuring the amplitude of the second signal, and storing a first initial calibration value associated with the first alternating signal and storing a second initial calibration value associated with the second alternating signal.

In a possible implementation form of the second aspect, the first signal has a first frequency, the second signal has a second frequency, the first frequency preferably equal to the second frequency, the first signal preferably being out of phase with the second signal.

In a possible implementation form of the second aspect, the biopotential signal produced by a person is an electromyography (EMG) or an electroencephalography (EEG) or an electrocardiography (ECG) signal.

In a possible implementation form of the first or second aspect, the biopotential signal produced by a person is a Biopotential Primer, Electrocardiography (ECG), Electrodermal Activity (EDA), Electroencephalography (EEG), Electrogastrography (EGG), Electromyography (EMG), Electrooculography (EOG), Impedance Cardiography (ICG) signal.

In a possible implementation form of the second aspect, the method comprising compensating for the effect of movement artifacts on the first electrode by adjusting the resistance of the first variable controlled resistance load, compensating for the effect of movement artifacts on the second electrode by adjusting the resistance of the second variable controlled resistance load.

In a possible implementation form of the second aspect, the method comprising compensating for the effect of sweat accumulation on the skin of a person by increasing the variable controlled gain.

In a possible implementation form of the second aspect, the method comprising adjusting the variable controlled gain as a function of a change in amplitude of the measured first signal and/or of the amplitude of the measured second signal when and only when the amplitude of both the first signal and the amplitude of the second signal changes simultaneously and in the same direction.

By providing an individual signal for the first and an individual signal for the second electrode, and by monitoring the signals individually, as well as relative to one another, it becomes possible to determine whether changes in impedance are caused by movement artifacts, the first electrode or the second electrode or both the first and second electrode or by sweat accumulation. With this insight, it becomes possible to provide an apparatus that can accurately compensate for impedance changes caused by movement artifacts and accurately compensate for signal loss due to acclamation sweat.

When detecting an individual signal change for the first or second signal alone, generated by the first signal generator and the second signal generator it is possible to compensate for movement artifacts and electrode displacement by increasing or decreasing the variable controlled resistance load connected to the first or second electrode and the first or second signal generator, respectively. When detecting a mismatch of skin electrode interface with respect to the reference electrode, the amplitude of both signals changes simultaneously and it becomes possible to compensate for sweat accumulation by increasing the variable controlled gain of the third amplifier 118.

In a possible implementation of the second aspect, the first signal has a first frequency, the second signal has a second frequency, the first frequency preferably being equal to the second frequency, and the first signal preferably being out of phase with the second signal.

In a possible implementation of the second aspect, the method comprises compensating for the effect of movement artifacts on the first electrode by adjusting the resistance of the first variable controlled resistance load and compensating for the effect of movement artifacts on the second electrode by adjusting the resistance of the second variable controlled resistance load.

In a possible implementation form of the second aspect comprising for the effect of sweat accumulation on the skin of a person, by increasing the variable controlled gain.

In a possible implementation form of the second aspect, the signal injected by the first and second signal generators may be an electrical pulse or square waves or triangle waves with known time-difference components prior to transmitting the signal.

These and other aspects will be apparent from and the embodiment(s) described below.

### BRIEF DESCRIPTION OF THE DRAWINGS

In the following detailed portion of the present disclosure, the aspects, embodiments, and implementations will be explained in more detail with reference to the example embodiments shown in the drawings, in which:
Fig. 1 is a diagrammatic representation of the apparatus in accordance with an embodiment;
Fig. 2 and Fig. 11 is a graph showing an example of the apparatus detecting and compensating for impedance changes caused by movement artifacts over time in a simulation;
Fig. 3 is a graph showing an example of the apparatus detecting and correcting for signal amplitude attenuation caused by sweat buildup on the skin over time in a simulation;
Fig. 4 is a graph showing the attenuation of a frequency during sweat over time in a simulation;
Fig. 5 is a graph showing the attenuation of a signal during electrode displacement over time in a simulation.
Fig. 6 is a flowchart overview of the method for detecting and compensating for movement artifacts and sweat accumulation and compensation.
Fig. 7 is a flowchart of the calibration of the apparatus for the detection and compensation of movement artifacts and sweat.
Fig. 8 and 9 are flowchart representation of a movement artifact detection and compensation
Fig. 10 is a flowchart of a sweat accumulation detection and compensation;
Fig. 12 is a diagrammatic representation of the apparatus in accordance with an embodiment.

### DETAILED DESCRIPTION

The apparatus and method described herein with reference to allow for the short-or long-term monitoring of a biopotential signal of a patient.

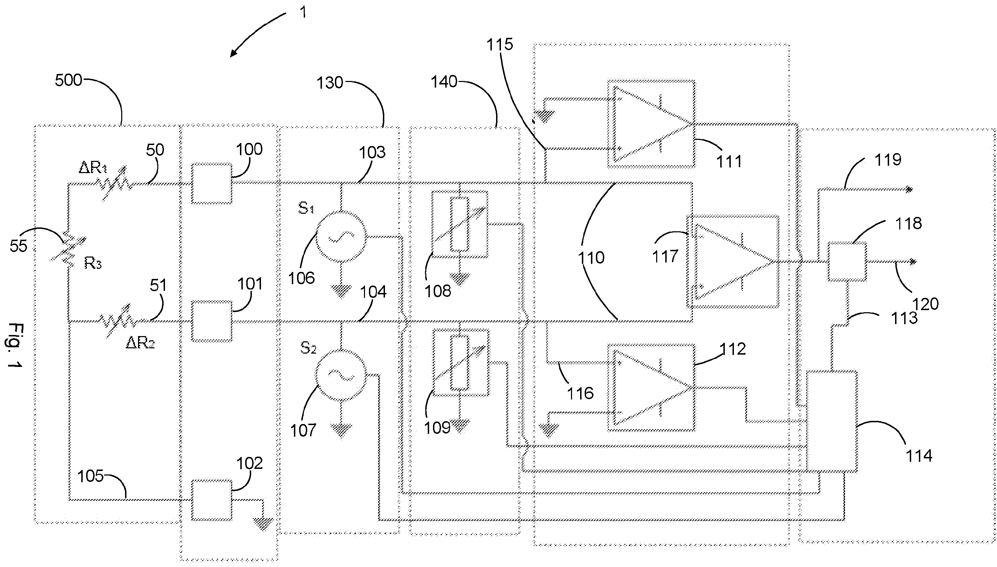

Fig. 1 is a diagrammatic representation of the electric diagram of the apparatus 1. A first electrode 100, a second electrode 101 and a reference electrode 102 for spaced placement on a subject's skin 500 are associated with the apparatus 1. The apparatus 1 has a first terminal 103 for connection to the first electrode 100, a second terminal 104 for connection to the second electrode 101 and a third terminal 105 for connection to the reference electrode 102.

The first electrode 100, the second electrode 101 and/or the reference electrodes 102 are preferably dry surface electrodes.

A variable controlled signal generator system 130 is connected to the first 100 and second electrode 101. A first signal generator 106 is connected to the first electrode 100 for generating a first alternating signal and a second signal generator 107 is connected to the second electrode 101 for generating a second alternating signal, preferably out-of-phase with the first alternating signal generated by the first signal generator 106.

The first alternating signal and the second alternating signal utilizes an alternating current with a frequency in the range of 100 to 700, preferably 500 Hz. It is important to note that the frequency of 500 Hz or higher and the implementation of input loads does not have any effect on the measured biopotential signal. The first alternating signal and the second alternating signals can have the same frequency but are out-of-phase relative to each other. The first and second signals may be 40° to 320° out of phase relative to each other, preferably the first and second signals are 120° out of phase relative to each other. Initially, the first injected signal and the second injected signal serve as reference signals for calibration measurements and are stored as calibrated values.

An input impedance compensation load system 140 for compensation of movement artifacts is connected to the first and second terminals 103 and 104. The impedance compensation load system 140 comprises a first variable controlled resistance load 108 connected to the first terminal 103 and a second variable controlled resistance load 109 connected to the second terminal 104 and is used in connection with impedance changes caused by movement artifact compensation.

A first circuitry 110 is configured to measure the biopotential signal from the first 103 and second 104 terminals. The first circuitry 110 comprises an instrumental amplifier 117 connected to the first 103 and second 104 terminals for amplifying the biopotential signal.

A second circuitry 115 is connected to the first terminal 103 for sensing and/or amplifying the amplitude of the first signal generated by the first signal generator 106. The second circuitry 115 comprises a first amplifier 111, preferably an operational amplifier configured to amplify the amplitude of the first signal.

A third circuitry 116 is connected to the second terminal 104 for sensing and/or amplifying the amplitude of the second signal generated by the second signal generator 107. The third circuitry 116 comprises a second amplifier 112, preferably an operational amplifier configured to amplify the amplitude of the second signal.

A fourth circuitry 113 is connected to the output of the first circuitry 110 and is configured to apply a variable controlled gain to the output signal of the first circuitry 110. The fourth circuitry 113 comprises a third amplifier 118, preferably a digitally controlled amplifier connected to the output of the instrumental amplifier 117, and is configured to apply a variable controlled gain to the output signal of the instrumental amplifier 117 and is used in relation with the compensation for sweat accumulation on the person's skin and the calibration and re-calibration of the apparatus 1 during measurements.

The instrumental amplifier 117 produces a raw and non-sweat compensated analog output 119. The fourth circuitry 113 produces a raw and sweat-compensated analog output 120.

A controller 114 (such as e.g. a microprocessor) receives the output signals of the second circuitry 115 and the third circuitry 116. The controller 114 is configured to control and/or adapt the first variable controlled resistance load 108 as a function of the amplitude of the output signal of the second circuitry 115, to control and/or adapt the second variable controlled resistance load 109 as a function of the amplitude of the output signal of the third circuitry 116 and to control and/or adjust the variable controlled gain as a function of the amplitude of the output signal of the second circuitry 115 and/or of the amplitude of the output signal of the third circuitry 116 when, and only when the amplitude of both the output signal of both the second and third circuitry 115 and 116 change simultaneously and in the same direction. Increased skin-electrode resistance 50,51 caused by movement artifacts, is detected by the controller 114 as an increase in the respective output signal of the first or second amplifier 111 or 112, respectively.

The controller 114 adjusts the variable resistance loads in response to the signal received from the output signal of the first or second amplifier 111 or 112, respectively. The controller 114 in receipt of the signal data from output signal of the first or second amplifier 111 or 112, respectively is configured to increase or decrease the resistance load of the first or second variable resistances 108 or 109, respectively, when the signal from the second circuitry 115 or third circuitry 116 attenuates when compared to the initially recorded calibration value plus a tolerance of the second 115 and third circuitry 116. Thus, the controller 114 is configured to counter the effect of increase or decrease of the signal of the first circuitry 110 and the effect of increase or decrease of the signal of the second circuitry 115 and the third circuitry 116.

The controller 114 is configured to increase the variable controlled gain 118 when and only when the amplitude of both the output signals of the second and third circuitry 115 and 116 attenuate simultaneously and preferably with the same rate to compensate for sweat accumulation and the controller 114 is configured to update the calibrated values to new calibrated values (re-calibration of the apparatus 1) in order to have a new reference point of comparison. The controller 114 is configured to decrease the variable controlled gain 118 when and only when the amplitude of both the output signals of the second and third circuitry 115 and 116 increase simultaneously.

The controller 114 is configured to receive the signal outputs from the first amplifier 111 to control the first variable controlled resistance load 108 and the controller 114 is configured to receive the signal outputs from the second amplifier 112 to control the second variable controlled resistance load 109. The controller 114 is also configured to control the first signal generator 106 and second signal generator 107. The controller 114 is further configured to control the third amplifier either by hardware means or by using a software.

The apparatus 1 measures and stores the value of the first and second control signals and each serve as an initial reference calibrated value. The controller 114 determines a mismatch of the first 100 or second 101 skin electrode to skin interface with respect to the reference electrode 102 and the initial reference calibrated values.

At startup, the apparatus 1 measures the first and second signals and stores these values as initial calibration values, and determines the mismatch of skin electrode interface with respect to the reference electrode 102 and the initial calibration values.

The apparatus 1 determines accumulation of sweat on the skin 500 which causes shunting between the first 100 and second 101 electrodes by measuring the amplitude of the first signal and amplitude of the second signal and by detecting simultaneous changes of the first and second signal, e.g. a simultaneous decrease of the amplitude of the first and second signal plus a tolerance occurring at the same time. The attenuated amplitude of the measured biopotential signal is then compensated for by increasing the gain of the third amplifier 118, preferably by a factor corresponding to the amount of amplitude difference between the initial calibration values and the current amplitude values. The initially calibrated values are also re-calibrated with new values in order to keep track of the accumulated sweat over a period of time and ensure accurate measurements.

The variation of the amplitude control signal translates into movement artifacts and/or electrode displacements and the apparatus 1 increases or decreases the load system to compensate for electrode displacements. The apparatus 1 can also detect which electrode is displaced and correct for a single or multiple electrode displacement by increasing the load connected to the displaced electrode 100, 101.

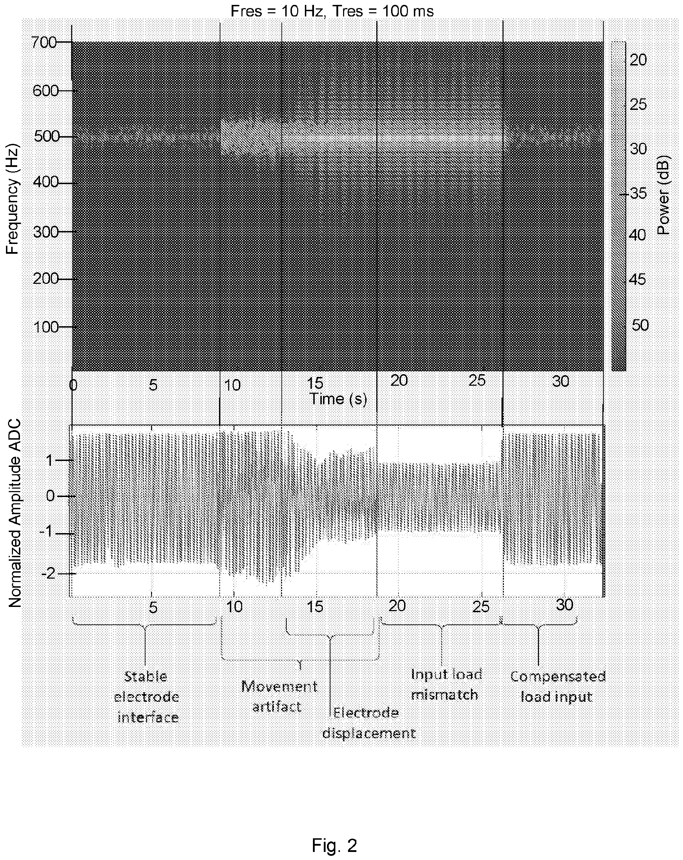

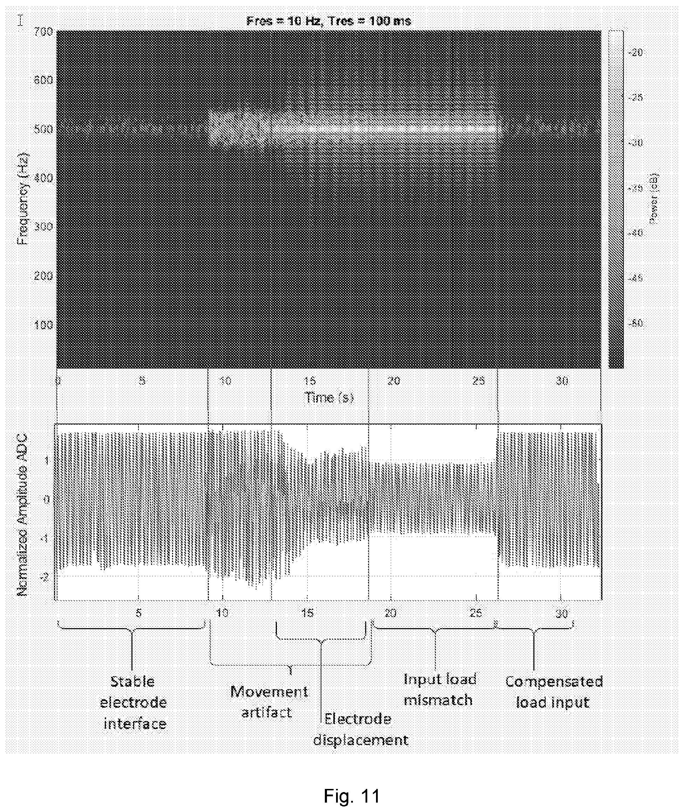

Fig. 2 and Fig. 11 (color-version of Fig. 2) is a graph showing an example of the apparatus 1 detecting and correcting for impedance changes caused by movement artifacts. When the electrodes 100 and 101 are placed onto a skin surface 500, there will initially be a stable electrode interface. As movement artifacts occur, the electrodes 100 and/or 101 and will be (temporarily) displaced as the active user movement will create a temporary gap between the skin 500 and the recording electrode 100 and/or 101 which results in a voltage spike in the EMG output. It is essential to compensate for the electrode displacement over time as these may be mistaken for actual muscle activation events during a measurement. The simulation shows the two out-of-phase driver frequencies set to be 500 Hz as it was found to be the most effective frequency for the accurate measurements over time. However, it is understood that the driver frequencies could any suitable value that is higher than 500 Hz.

Due to amplitude changes being detected in the generated signal, a movement artifact and electrode displacement is detected by the controller 114 and registered as an input load mismatch. The controller 114 adapts and adjusts the variable controlled resistance loads 108 and 109 to compensate for the movement artifacts and the signal is restored to that of, or close to that of before the movement artifact caused electrode displacement. As soon as the movement artifact ends, the controller 114 will re-adjust the respective variable controlled resistance loads 108, 109 to their previous values.

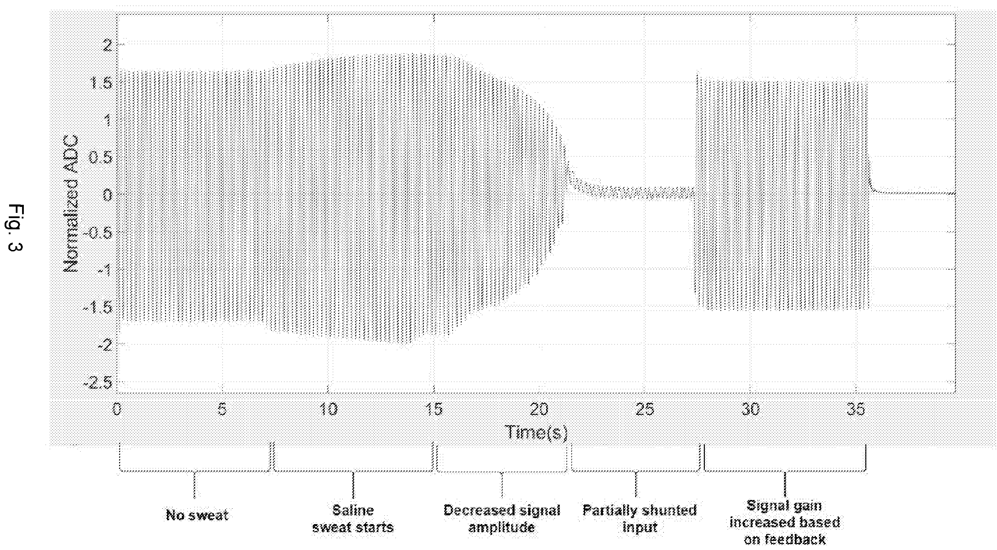

Fig. 3 shows an example of the apparatus 1 detecting and correcting for impedance changes caused by sweat in accordance with an embodiment. Sweat typically starts accumulating on the skin's surface within approximately 7 to 20 minutes of placing the electrodes 100 and 101 onto the skin surface 500 however this may be dependent on the individual, and depends on whether or not a sleeve or the like is used to keep the electrodes on the skin of the person. After placing the electrodes 100 and 101 onto the skin's surface, the first and second controlled variable loads 108 and 109 are set to a medium value, and the first signal generator 106 injects a signal to the first electrode 100 and the second signal generator 107 injects a second signal to the second electrode 101, the second signal preferably being out-of-phase with the first signal and an initial calibration value is recorded by measuring the output of the second circuitry 115 and the output of the third circuitry 116. The medium range for the resistor loads may be 5 MΩ in case the full range of the resistor load is 10 MΩ. As the saline sweat starts accumulating, the signal amplitude gradually decreases for both first and second signals due to shunting between the first 100 and second 101 electrode, reducing the strength of the output signal and the accuracy of biopotential signal measurements. The apparatus 1 compensates for this signal amplitude attenuation of both the first and second signal via the controller 114 either by hardware increasing the gain or using a digital software gain amplification to increase the gain. The gain may be increased back to that of, or close to that of before the sweat accumulation if and when the controller 114 registers a simultaneous increase in the first and second signals, to produce stable, accurate biopotential signal measurements. Upon a gain adjustment, the controller 114 adjusts the calibration values by increasing the gain by the magnitude difference of the initial calibration values and the new values, and both the initial and the later used re-calibrated values are stored. The signal gain is hence increased on feedback and the shunting effect of sweat accumulation is compensated for.

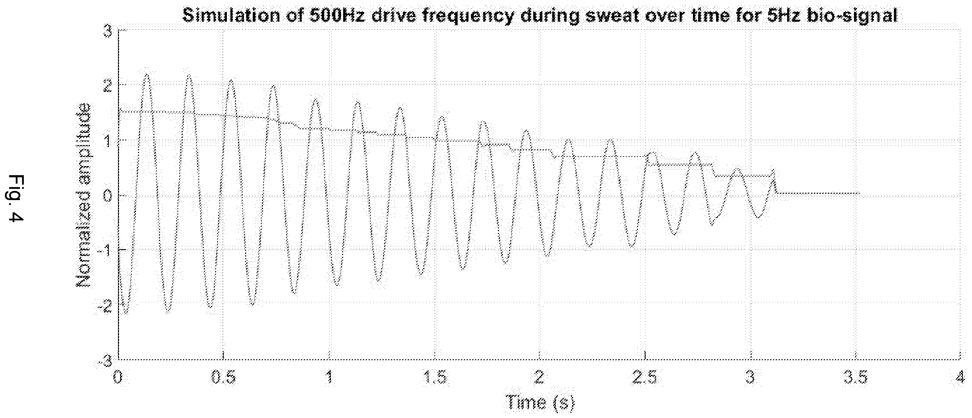

Fig. 4 shows the decrease of normalized amplitude signal due to sweat and its shunting effect without compensation, resulting in a compromised biopotential signal measurement in a simulation. The simulation shows the shunting effect of sweat accumulation over time for a 5 Hz biopotential signal. The normalized amplitude of the signals generated by the first signal generator 106 and the second signal generator 107 gradually decrease over time and the detected biopotential signal gradually decreases to the point where the biopotential signal can no longer be detected and the signal strength significantly decrease to the point of inaccurate measurements. This effect is overcome by the compensation system outlined above.

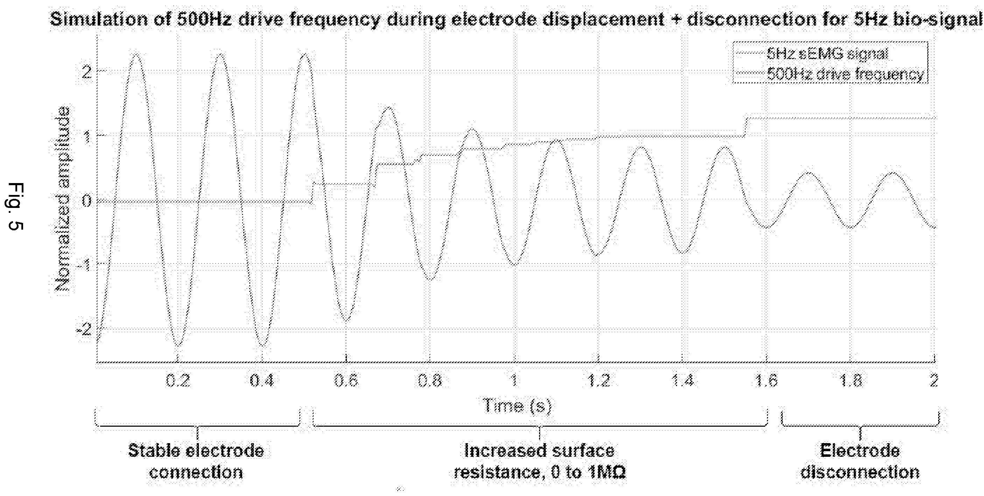

Fig. 5 shows a simulation of the attenuation of a frequency due to electrode displacement and movement artifacts over time without a compensation system outlined above. The stable electrode connection may be disturbed by movement artifacts which lead to increased surface resistance, the attenuation of the biopotential signal as well as the increase of the amplitude of the injected signal, leading to electrode disconnection and voltage spikes in the EMG measurement signals. The biopotential signal measured shows amplitude spikes and irregularities that are caused first by increased surface resistance and the consequent electrode displacements. The signal generated by the first signal generator 106 and the second signal generator 107 shows an irregular increase due to first the increased surface resistance and the lack of compensation for thereof and the complete electrode displacement and the loss of the original signal frequency. The apparatus 1 presents a solution for this problem.

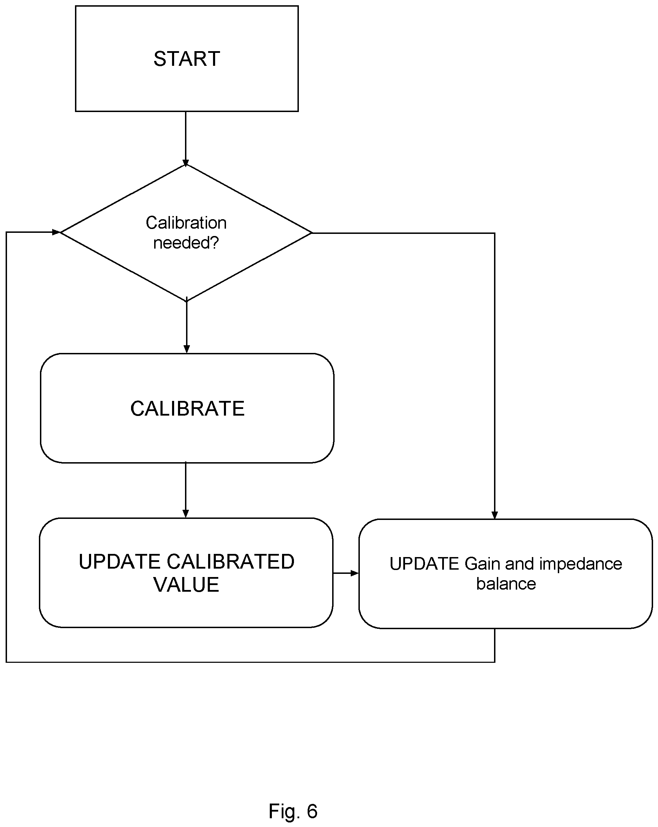

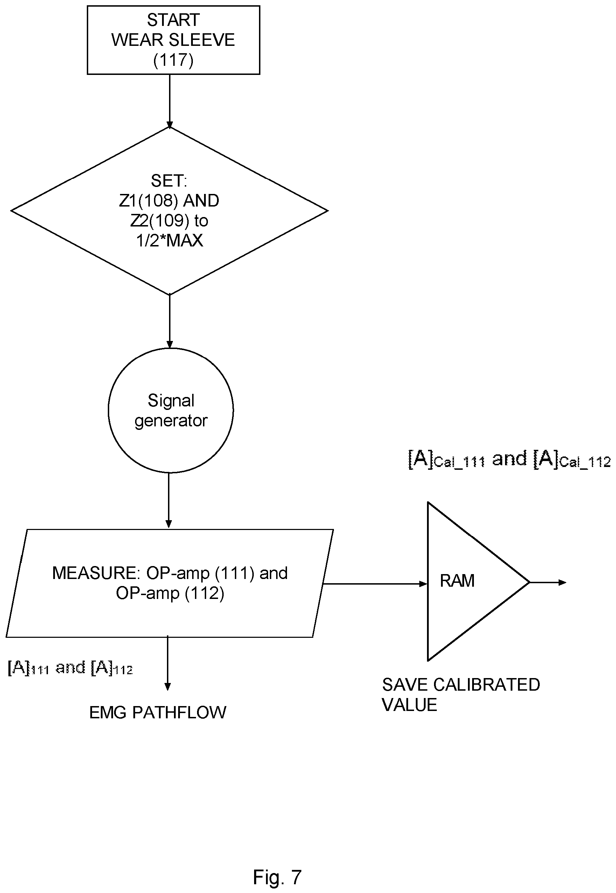

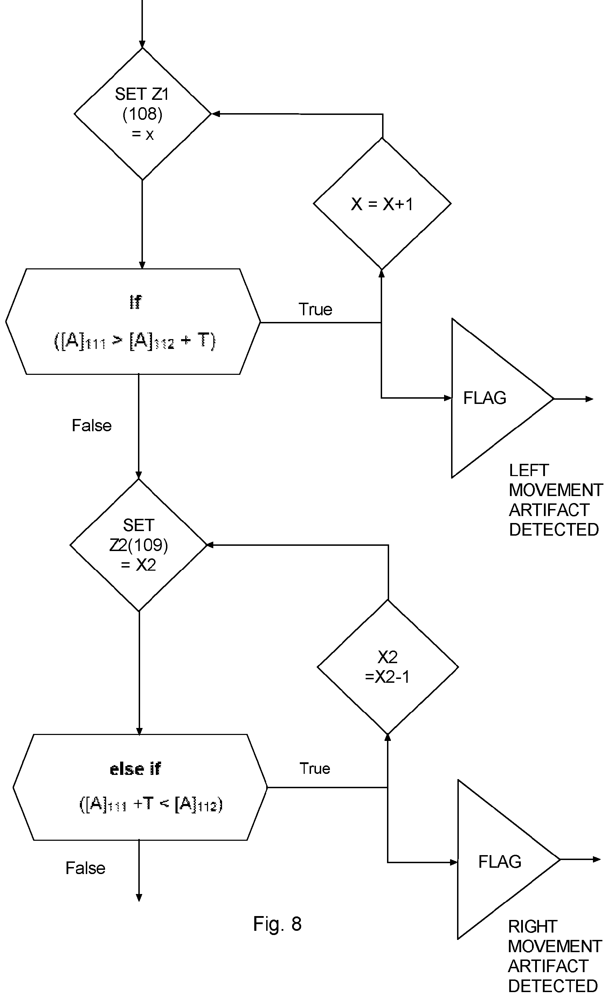

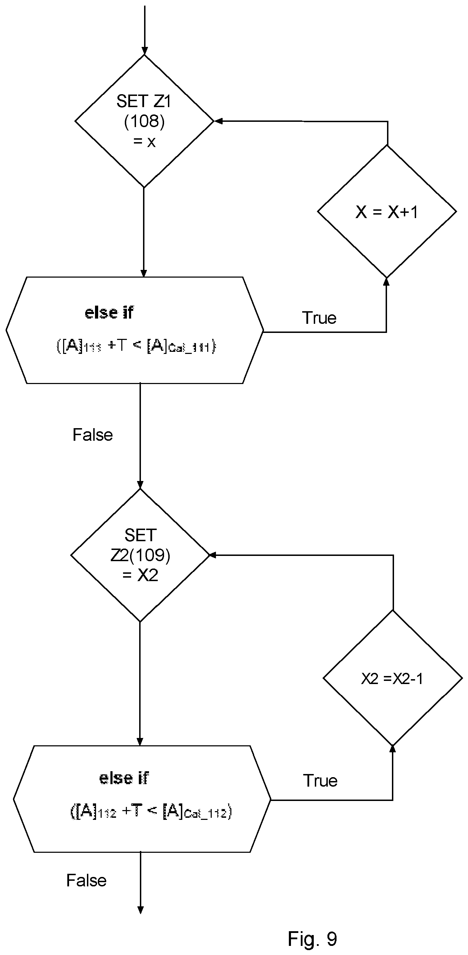

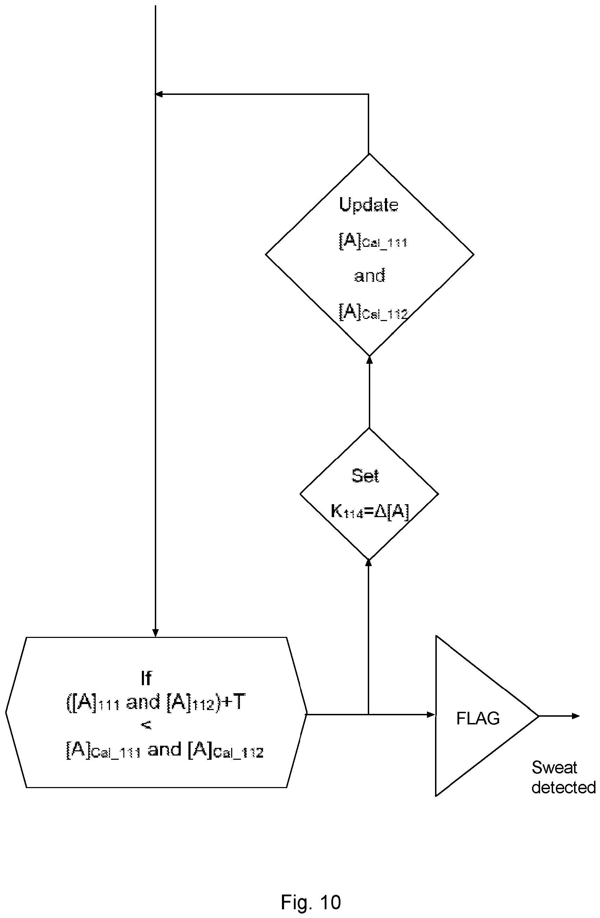

Fig. 6 is showing the overall method for detecting and compensating for movement artifacts and sweat accumulation, with Fig. 7, Fig. 8, Fig. 9 and Fig. 10 detailing each step outlined in Fig. 6. It is understood, that the detailed method begins in Fig 7. first showing the calibration of apparatus 1. Fig. 8 is understood to follow from the calibration steps of Fig. 7. Fig. 9 is understood to be a combination of Fig. 7 and Fig. 8. Fig. 10 is understood to be the final step of the detection and compensation method outlined in this application. It is understood that the order of the steps of the method may be interchangeable with each other.

Fig. 6 is a flowchart of the method for detecting and compensating for movement artifacts and sweat accumulation.

A first and second electrode 100 and 101 are placed onto the surface of the skin of a person and the system is calibrated with the initial calibration values recorded and updates. If no calibration is needed, e.g. as the initial values were recorded at an earlier stage, the apparatus 1 updates the gain and impedance balance upon registration of movement artifacts and/or sweat accumulation. This initiates a new feedback cycle and the system checks for the recorded calibration values and compares to those recorded in real-time, re-calibrating and updating the gain and impedance balance as required to compensate for movement artifacts and sweat accumulation.

Fig. 7 is a flowchart of the method for calibration of the apparatus 1 according to Fig. 1. After placing the first 100 and second 101 electrode onto the surface of the skin, the variable controlled loads 108 and 109 are set to a medium value plus a tolerance which is set to be between 0,0001% to 0,01% of the difference of the medium value, preferably 0,005% difference to the medium value. The first signal generator 106 applies a first alternating signal to the first electrode 100 and the second signal generator 107 applies a second alternating signal to the second electrode 101. The output of the second circuitry 115 and the third circuitry 116 is measured and the output values are recorded as initial calibration values for both the second circuitry 115 and the third circuitry 116.

Fig. 8 is a flowchart of the method for detecting and compensating for movement artifacts. After the initial calibration according to Fig. 7, the first variable controlled resistance load 108 is set to a medium value, plus a tolerance value which is set to 0,0001% to 0,01% of the medium value, preferably 0,005% of the medium value and the second variable controlled resistance load 109 is set to a medium value, plus a tolerance value which is set to 0,0001% to 0,01% of the medium value, preferably a 0,005% of the medium value. The controller 114 detects changes in the system with respect to the initial calibrated value.

If the output of the second circuitry 115 and the amplitude of the first amplifier 111 is higher than the output of the third circuitry 116 and the amplitude of the second amplifier 112 plus a tolerance value, the event is flagged as left movement artifact, and the first variable controlled resistance load 108 is increased, preferably stepwise in order to match the input load impedance of the first variable controlled load 108. The method will also check for the opposite condition.

If the output of the second circuitry 115 and the amplitude of the first amplifier 111, plus a tolerance, is lower than the output of the third circuitry 116 and the amplitude of the second amplifier 112, the event is flagged as right movement artifact, and the second variable controlled load 109 is increased, preferably stepwise in order to match the input load impedance of the second variable controlled load 109.

Else if the above condition proves false, the apparatus 1 according to Fig. 1 proceeds onto the next steps as outlined in Fig. 9 and 10.

Fig. 9 is a flowchart representation of a movement artifact detection and compensation. The flowchart is understood to be a combination of the previous steps outlined in Fig. 8.

If the output of the second circuitry 115 and the amplitude of the first amplifier 111, plus a tolerance, is lower than the initial calibrated value of the third circuitry 116 and the amplitude of the second amplifier 112, the event is flagged and the first variable controlled resistance load 108 is decreased, preferably stepwise, in order to match the input load impedance of the first variable controlled load 108.

If the output of the third circuitry 116 and the amplitude of the second amplifier 112, plus a tolerance, is lower than the initial calibrated value of the third circuitry 116 and the amplitude of the second amplifier 112, the event is flagged and the second variable controlled resistance load 109 is decreased, preferably stepwise, in order to match the input load impedance of the second variable controlled load 109.

If the condition is false, the controller 114 checks for sweat accumulation.

Fig. 10 is a flowchart of sweat accumulation detection and compensation. The flowchart is understood to continue from the previous steps as outlined in Fig. 8.

If the output of the second circuitry 115 and the amplitude of the first amplifier 111 and the output of the third circuitry 116 and the amplitude of the second amplifier 112, plus a tolerance, is lower than, and/or a simultaneous decrease and/or decrease with the same rate the initial calibrated value of the second circuitry 115 and the amplitude of the first amplifier and the initial calibrated value of the third circuitry 116 and the amplitude of the second amplifier 112, the event is flagged as sweat accumulation.

The controller 114 then adjusts the variable gain e.g. by increasing the gain via the amplitude differences between the newly recorded and the initial calibration value. The controller 114 also updates the calibrated values with the new, re-calibrated values that are equal to the amplitude difference between the newly recorded value and the initial calibration value and saves all values to keep track of the sweat accumulation and related changes to the calibrated and re-calibrated values due to sweat accumulation.

The method will then repeat the feedback loop cycle.

The apparatus 1 and method simultaneously detect signal deterioration caused by sweat and movement artifacts, while also compensating for these effects to allow for short-or long-term monitoring of biopotential signals of a person, such as e.g. a patient with a condition.

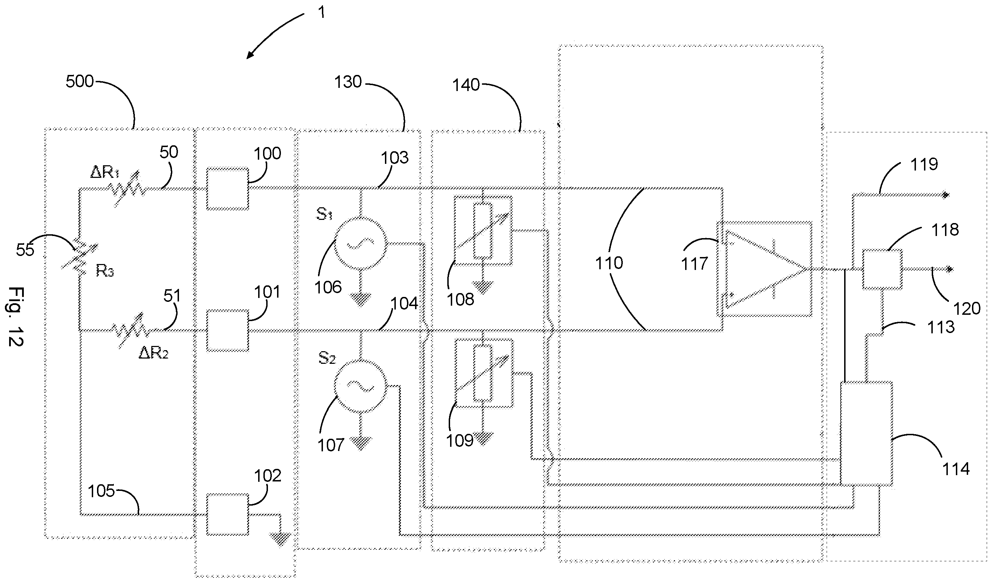

Fig. 12 is a diagrammatic representation of the apparatus in accordance with an embodiment. It is understood that the same numbering has been used for the same constituents of the apparatus as in Fig. 1 and detailed above. According to this embodiment, the apparatus comprises only one amplifier 117 when a microprocessor 114 is employed that is powerful enough and configured for performing real-time analysis in the time-frequency domain (e.g. to perform a Fast Fourier Transformation (FFT)). The feedback connection between the amplifier 117 and the microprocessor 114 allows for the omission of amplifiers 111, 112, as the analog output of the amplifier 117 generates a feed to the microprocessor input 114, and the (powerful) microprocessor 114 is configured to perform a FFT on this signal to thereby distinguish between the portion of the signal originating from the first signal generator 106 and the portion of the signal originating from the second signal generator 107, respectively. This allows the microprocessor 114 to analyze and differentiate between the origin of the output signals of the first circuitry for controlling the variable resistance loads 108,109 individually and independently of each other.

The first 106 and second 107 signal generators provide a signal to the first 100 and second 101 electrodes, which is amplified by the amplifier 117 and is analyzed by the microprocessor 114. The first and second signals may have the same frequency but are out-of-phase relative to each other allowing for easy differentiation between the two signals. The controller 114 is configured for detecting the output signals of the first circuitry 110 characteristic of the first or second alternating signal and is then able to determine the amplitude of the first and second alternating signals. The controller 114, in receipt of the amplitude of the first and second alternative signals, is then capable of controlling and adapting the first and second variable resistance loads 108, 109 to compensate for signal attenuation and is further capable of adjusting the variable controlled gain 118 as a function of the determined amplitude of the first and second alternating signal, hence effectively overcoming the attenuation effect caused by movement artifacts or sweat accumulation.

The various aspects and implementations have been described in conjunction with various embodiments herein. However, other variations to the disclosed embodiments can be understood and effected by those skilled in the art in practicing the claimed subject-matter, from a study of the drawings, the disclosure, and the appended claims. In the claims, the word "comprising" does not exclude other elements or steps, and the indefinite article "a" or "an" does not exclude a plurality. A single controller or processor or other unit may fulfill the functions of several items recited in the claims. The mere fact that certain measures are recited in mutually different dependent claims does not indicate that a combination of these measured cannot be used to advantage.

The reference signs used in the claims shall not be construed as limiting the scope. Unless otherwise indicated, the drawings are intended to be read (e.g., cross-hatching, arrangement of parts, proportion, degree, etc.) together with the specification, and are to be considered a portion of the entire written description of this disclosure.

## Claims

1. An apparatus (1) for sensing a biopotential signal on the skin (500) of a person using at least a first skin electrode (100), a second skin electrode (101) and a reference skin electrode (102) for spaced placement on said skin (500) of said person, the apparatus (1) comprising:
- a first terminal (103) for connection to said first electrode (100),
- a second terminal (104) for connection to said second electrode (101),
- a first circuitry (110) configured for measuring the biopotential signal from said first (103) and said second (104) terminal,
- a third terminal (105) for connection to said reference skin electrode (102),
- a first variable controlled resistance load (108) connected to said first terminal (103),
- a second variable controlled resistance load (109) connected to said second terminal (104),
- a first signal generator (106) for generating a first alternating signal connected to said first terminal (103),
- a second signal generator (107) for generating a second alternating signal connected to said second terminal (104),
- a fourth circuitry (113) connected to the output of said first circuitry (110) and configured to apply a variable controlled gain (118) to the output signal of said first circuitry (110),
- a controller (114) in receipt of:
• an output signal of said first circuitry (110),
• a signal comprising a component representative of the amplitude of said first alternating signal,
• a signal comprising a component representative of the amplitude of said second alternating signal,
the controller (114) being configured:
• to determine the amplitude of said first alternating signal,
• to determine the amplitude of said second alternating signal,
• to control said first variable controlled resistance load (108) and said second variable controlled resistance load (109),
• to control said variable controlled gain (118),
• to adapt the load of said first variable controlled resistance load (108) as a function of a change in the determined amplitude of said first alternating signal,
• to adapt the load of said second variable controlled resistance load (109) as a function of a change in the determined amplitude of said second alternating signal, and
• to adjust said variable controlled gain (118) as a function of the determined amplitude of said first alternating signal and of the determined amplitude of said second alternating signal, when, and only when, the determined amplitude of both said first alternating signal and said second alternating signal change simultaneously.

2. The apparatus according to claim 1, wherein the signal comprising at least a component representative of the amplitude of said first alternating signal and the signal comprising a component representative of the amplitude of said second alternating signal is the output signal of said first circuitry (110).

3. The apparatus according to claim 1 or 2, wherein the controller being configured:
- to perform a time-frequency analysis of the output signal of said first circuitry (110) and thereby determine the portion of the output signal of said first circuitry (110) originating from said first alternating signal and the portion of the output signal of said first circuitry (110) originating from said second alternating signal,
- to determine the amplitude of the output signal of said first circuitry (110) originating from said first alternating signal,
- to determine the amplitude of the output signal of said first circuitry (110) originating from said second alternating signal,
- to control said first variable controlled resistance load (108),
- to control said second variable controlled resistance load (109),
- to control said variable controlled gain (118),
- to adapt the load of said first variable controlled resistance load (108) as a function of the amplitude of the output signal of said first circuitry (110) originating from said first alternating signal,
- to adapt the load of said second variable controlled resistance load (109) as a function of the amplitude of the output signal of said first circuitry (110) originating from said second alternating signal, and
- to adjust said variable controlled gain (118) as a function of the amplitude of the output signal of said first circuitry (110) originating from said first alternating signal and of the amplitude of the output signal of said first circuitry (110) originating from said second alternating signal when, and only when, the amplitude of both the output signal of said first circuitry (110) originating from said first alternating signal and the amplitude of the output signal of said first circuitry (110) originating from said second alternating signal change simultaneously.

4. The apparatus (1) according to claim 1 to 3, comprising a second circuitry (115) connected to said first terminal (103) for sensing and/or amplifying the amplitude of said first signal and a third circuitry (116) connected to said second terminal (104) for sensing and/or amplifying the amplitude of said second signal, a controller (114) in receipt of the output signal of said second circuitry (115) and in receipt of the output signal of said third circuitry (116), said controller (114) being configured:
- to adapt the load of said first variable controlled resistance load (108) as a function of the amplitude of said output signal of said second circuitry (115),
- to adapt the load of said second variable controlled resistance load (109) as a function of the amplitude of said output signal of said third circuitry (116), and
- to adjust said variable controlled gain (118) as a function of the amplitude of said output signal of said second circuitry (115) and of the amplitude of the output signal of the third circuitry (116) when, and only when, the amplitude of both the output signal of both said second circuitry (115) and said third circuitry (116) change simultaneously.

5. The apparatus (1) according to claim 1 to 4, wherein said controller (114) is configured to:
A) increase said variable controlled gain (118) when and only when the amplitude of both the output signal of said second (115) and said third (116) circuitry attenuate simultaneously, and/or
B) decrease said variable controlled gain (118) when and only when the amplitude of both the output signal of the second (115) and third (116) circuitry increase simultaneously, and/or
C) set the first variable controlled resistance load (108) and the second variable controlled resistant load (109) both to a medium value, and that the controller (114) is configured to thereupon calibrate the apparatus as a function of the amplitude of the output signal of the second circuitry (115) and as a function of the output signal of the third circuitry (116), and wherein the controller (114) preferably is configured to store a first initial calibration value associated with the first alternating signal and preferably is configured to store a second initial calibration value associated with the second alternating signal.

6. The apparatus (1) according to claim 5 option C), wherein the controller (114) is configured to update the first and second initial calibration value after the controller has adjusted the variable controlled gain (118).

7. The apparatus (1) according to any one of claims 1 to 3, wherein the first circuity (110) comprises an instrumental amplifier (117) connected to the first (103) and second (104) terminal for amplifying the biopotential signal.

8. The apparatus (1) according to any one of claims 1 to 5, wherein the second circuitry (115) comprises a first amplifier (111) connected to the first terminal (103) for amplifying the amplitude of the first signal, the first amplifier (111) preferably being an operational amplifier, or
wherein the third circuitry (116) comprises a second amplifier (112) connected to the second terminal (104) for amplifying the amplitude of the second signal, the second amplifier (112) preferably being an operational amplifier.

9. The apparatus (1) according to any one of claim 1 to 6, wherein the fourth circuitry (113) comprises digitally controlled amplifier (118) connected to the output of the instrumental amplifier (117) and configured to apply a variable controlled gain to the output signal of the instrumental amplifier (117).

10. The apparatus (1) according to claim 9, wherein the controller (114) is in receipt of the output signal of the first amplifier (111) and in receipt of the output of the second amplifier (112),
the controller (114) being configured to increase the resistance of the first variable controlled resistance load (108) when the amplitude of the output of the first amplifier (111) decreases and configured to decrease the resistance of the first variable controlled resistance load (108) when the amplitude of the output of the first amplifier (111) increases, and
the controller (114) being configured to increase the resistance of the second variable controlled resistance load (109) when the amplitude of the output of the second amplifier (112) decreases and configured to decrease the resistance of the second variable controlled resistance load (109) when the amplitude of the output of the second amplifier (112) increases.

11. The apparatus (1) according to claim 1 to 10, wherein the first (100), the second (101) and/or the reference electrodes (102) are dry surface electrodes and/or, wherein the first signal has a first frequency, the second signal has a second frequency, the first frequency preferably being equal to the second frequency, and the first signal preferably being out of phase with the second signal.

12. A method for sensing a biopotential signal on the skin (500) of a person, the method comprising:
placing a first skin electrode (100), a second skin electrode (101) and a reference skin electrode (102) on the skin (500) of the person,
applying a first alternating signal to the first electrode (100),
connecting a first variable controlled resistance load (108) to the first electrode (100),
connecting a second variable controlled resistance load (109) to the second electrode (101),
measuring the amplitude of the first signal and adjusting the resistance of the first variable controlled resistance load (108), as a function of a change in amplitude of the first signal,
applying a second alternating signal to the second electrode (101),
measuring the amplitude of the second signal and adjusting the resistance of the second variable controlled resistance load (109) as a function of a change in amplitude of the second signal,
measuring the biopotential signal with the first (100) and second (101) electrode and applying a variable controlled gain to the measured biopotential signal, and
adjusting the variable controlled gain (118) as a function of a change in amplitude of the measured first signal and of the amplitude of the measured second signal when and only when the amplitude of both the first signal and the amplitude of the second signal changes simultaneously.

13. The method according to claim 12, wherein setting the first variable controlled resistance load (108) and the second variable controlled resistance load (109) to approximately half the maximum of the fully operational range of the respective variable controlled resistance load and measuring the amplitude of the first signal and a measuring the amplitude of the second signal, and storing a first initial calibration value associated with the first alternating signal and storing a second initial calibration value associated with the second alternating signal.

14. The method according to claim 12, wherein the first signal has a first frequency, the second signal has a second frequency, the first frequency preferably being equal to the second frequency, and the first signal preferably being out of phase with the second signal, and/or
wherein the biopotential signal produced by the person is an electromyography (EMG) or an electroencephalography (EEG) or an electrocardiography (ECG) signal.

15. The method according to any one of claims 12 to 14, comprising compensating for the effect of:
A) movement artifacts on the first electrode (100) by adjusting the resistance of the first variable controlled resistance load (108), compensating for the effect of movement artifacts on the second electrode (101) by adjusting the resistance of the second variable controlled resistance load (109) and/or
B) sweat accumulation on the skin of the person, by increasing the variable controlled gain (118).

## Patentansprüche

1. Vorrichtung (1) zum Erfassen eines Biopotenzialsignals auf der Haut (500) einer Person unter Verwendung von mindestens einer ersten Hautelektrode (100), einer zweiten Hautelektrode (101) und einer Referenzhautelektrode (102) zur beabstandeten Platzierung auf der Haut (500) der Person, wobei die Vorrichtung (1) Folgendes umfasst:
- einen ersten Anschluss (103) zur Verbindung mit der ersten Elektrode (100),
- einen zweiten Anschluss (104) zur Verbindung mit der zweiten Elektrode (101),
- einen ersten Schaltkreis (110), der zum Messen des Biopotenzialsignals von dem ersten (103) und dem zweiten (104) Anschluss konfiguriert ist,
- einen dritten Anschluss (105) zur Verbindung mit der Referenzhautelektrode (102),
- einen ersten Verbraucher (108) mit variablem kontrolliertem Widerstand, der mit dem ersten Anschluss (103) verbunden ist,
- einen zweiten Verbraucher (109) mit variablem kontrolliertem Widerstand, der mit dem zweiten Anschluss (104) verbunden ist,
- einen ersten Signalgenerator (106) zum Erzeugen eines ersten Wechselsignals, der mit dem ersten Anschluss (103) verbunden ist,
- einen zweiten Signalgenerator (107) zum Erzeugen eines zweiten Wechselsignals, der mit dem zweiten Anschluss (104) verbunden ist,
- einen vierten Schaltkreis (113), der mit dem Ausgang des ersten Schaltkreises (110) verbunden und dazu konfiguriert ist, eine variable kontrollierte Verstärkung (118) an das Ausgangssignal des ersten Schaltkreises (110) anzulegen,
- eine Steuerung (114), die Folgendes empfangen kann:
• ein Ausgangssignal des ersten Schaltkreises (110),
• ein Signal, das eine Komponente umfasst, die die Amplitude des ersten Wechselsignals angibt,
• ein Signal, das eine Komponente umfasst, die die Amplitude des zweiten Wechselsignals angibt,
- wobei die Steuerung (114) zu Folgendem konfiguriert ist:
• Bestimmen der Amplitude des ersten Wechselsignals,
• Bestimmen der Amplitude des zweiten Wechselsignals,
• Steuern des ersten Verbrauchers (108) mit variablem kontrolliertem Widerstand und des zweiten Verbrauchers (109) mit variablem kontrolliertem Widerstand,
• Steuern der variablen kontrollierten Verstärkung (118),
• Anpassen der Last des ersten Verbrauchers (108) mit variablem kontrolliertem Widerstand in Abhängigkeit von einer Veränderung der bestimmten Amplitude des ersten Wechselsignals,
• Anpassen der Last des zweiten Verbrauchers (109) mit variablem kontrolliertem Widerstand in Abhängigkeit von einer Veränderung der bestimmten Amplitude des zweiten Wechselsignals und
• Einstellen der variablen kontrollierten Verstärkung (118) in Abhängigkeit von der bestimmten Amplitude des ersten Wechselsignals und der bestimmten Amplitude des zweiten Wechselsignals, wenn und nur wenn sich die bestimmte Amplitude sowohl des ersten Wechselsignals als auch des zweiten Wechselsignals gleichzeitig verändern.

2. Vorrichtung nach Anspruch 1, wobei das Signal, das mindestens eine Komponente umfasst, die die Amplitude des ersten Wechselsignals angibt, und das Signal, das eine Komponente umfasst, die die Amplitude des zweiten Wechselsignals angibt, das Ausgangssignal des ersten Schaltkreises (110) ist.

3. Vorrichtung nach Anspruch 1 oder 2, wobei die Steuerung zu Folgendem konfiguriert ist:
- Durchführen einer Zeit-Frequenz-Analyse des Ausgangssignals des ersten Schaltkreises (110) und dadurch Bestimmen des Teils des Ausgangssignals des ersten Schaltkreises (110), das von dem ersten Wechselsignal stammt, und des Teils des Ausgangssignals des ersten Schaltkreises (110), das von dem zweiten Wechselsignal stammt,
- Bestimmen der Amplitude des Ausgangssignals des ersten Schaltkreises (110), das von dem ersten Wechselsignal stammt,
- Bestimmen der Amplitude des Ausgangssignals des ersten Schaltkrieses (110), das von dem zweiten Wechselsignal stammt,
- Steuern des ersten Verbrauchers (108) mit variablem kontrolliertem Widerstand,
- Steuern des zweiten Verbrauchers (109) mit variablem kontrolliertem Widerstand,
- Steuern der variablen kontrollierten Verstärkung (118),
- Anpassen der Last des ersten Verbrauchers (108) mit variablem kontrolliertem Widerstand in Abhängigkeit von der Amplitude des Ausgangssignal des ersten Schaltkreises (110), das von dem ersten Wechselsignal stammt,
- Anpassen der Last des zweiten Verbrauchers (109) mit variablem kontrolliertem Widerstand in Abhängigkeit von der Amplitude des Ausgangssignals des ersten Schaltkreises (110), das von dem zweiten Wechselsignal stammt, und
- Einstellen der variablen kontrollierten Verstärkung (118) in Abhängigkeit von der Amplitude des Ausgangssignals des ersten Schaltkreises (110), das von dem ersten Wechselsignal stammt, und der Amplitude des Ausgangssignal des ersten Schaltkreises (110), das von dem zweiten Wechselsignal stammt, wenn und nur wenn sich sowohl die Amplitude des Ausgangssignals des ersten Schaltkreises (110), das von dem ersten Wechselsignal stammt, als auch die Amplitude des Ausgangssignals des ersten Schaltkreises (110), das von dem zweiten Wechselsignal stammt, gleichzeitig verändern.

4. Vorrichtung (1) nach den Ansprüchen 1 bis 3, umfassend einen zweiten Schaltkreis (115), der mit dem ersten Anschluss (103) verbunden ist, zum Erfassen und/oder Verstärken der Amplitude des ersten Signals und einen dritten Schaltkreis (116), der mit dem zweiten Anschluss (104) verbunden ist, zum Erfassen und/oder Verstärken der Amplitude des zweiten Signals, eine Steuerung (114), die das Ausgangssignal des zweiten Schaltkreises (115) empfangen kann und das Ausgangssignal des dritten Schaltkreises (116) empfangen kann, wobei die Steuerung (114) zu Folgendem konfiguriert ist:
- Anpassen der Last des ersten Verbrauchers (108) mit variablem kontrolliertem Widerstand in Abhängigkeit von der Amplitude des Ausgangssignals des zweiten Schaltkreises (115),
- Anpassen der Last des zweiten Verbrauchers (109) mit variablem kontrolliertem Widerstand in Abhängigkeit von der Amplitude des Ausgangssignals des dritten Schaltkreises (116) und
- Anpassen der variablen kontrollierten Verstärkung (118) in Abhängigkeit von der Amplitude des Ausgangssignals des zweiten Schaltkreises (115) und der Amplitude des Ausgangssignals des dritten Schaltkreises (116), wenn und nur wenn sich die Amplitude des Ausgangssignals sowohl des zweiten Schaltkreises (115) als auch des dritten Schaltkreises (116) gleichzeitig verändern.

5. Vorrichtung (1) nach den Ansprüchen 1 bis 4, wobei die Steuerung (114) zu Folgendem konfiguriert ist:
A) Erhöhen der variablen kontrollierten Verstärkung (118), wenn und nur wenn sich die Amplitude des Ausgangssignals sowohl des zweiten (115) als auch des dritten (116) Schaltkreises gleichzeitig verringern, und/oder
B) Reduzieren der variablen kontrollierten Verstärkung (118), wenn und nur wenn sich die Amplitude des Ausgangssignals sowohl des zweiten (115) als auch des dritten (116) Schaltkreises gleichzeitig erhöhen, und/oder
C) Festlegen des ersten Verbrauchers (108) mit variablem kontrolliertem Widerstand und des zweiten Verbrauchers (109) mit variablem kontrolliertem Widerstand auf einen mittleren Wert, wobei die Steuerung (114) dazu konfiguriert ist, daraufhin die Vorrichtung in Abhängigkeit von der Amplitude des Ausgangssignals des zweiten Schaltkreises (115) und in Abhängigkeit von dem Ausgangssignal des dritten Schaltkreises (116) zu kalibrieren, und wobei die Steuerung (114) vorzugsweise dazu konfiguriert ist, einen ersten anfänglichen Kalibrierungswert, der dem ersten Wechselsignal zugeordnet ist, zu speichern und vorzugsweise einen zweiten anfänglichen Kalibrierungswert, der dem zweiten Wechselsignal zugeordnet ist, zu speichern.

6. Vorrichtung (1) nach Anspruch 5, Option C), wobei die Steuerung (114) dazu konfiguriert ist, den ersten und den zweiten anfänglichen Kalibrierungswert zu aktualisieren, nachdem die Steuerung die variable kontrollierte Verstärkung (118) eingestellt hat.

7. Vorrichtung (1) nach einem der Ansprüche 1 bis 3, wobei der erste Schaltkreis (110) einen Instrumentalverstärker (117), der mit dem ersten (103) und dem zweiten (104) Anschluss verbunden ist, zum Verstärken des Biopotenzialsignals umfasst.

8. Vorrichtung (1) nach einem der Ansprüche 1 bis 5, wobei der zweite Schaltkreis (115) einen ersten Verstärker (111), der mit dem ersten Anschluss (103) verbunden ist, zum Verstärken der Amplitude des ersten Signals umfasst, wobei der erste Verstärker (111) vorzugsweise ein Operationsverstärker ist, oder
wobei der dritte Schaltkreis (116) einen zweiten Verstärker (112), der mit dem zweiten Anschluss (104) verbunden ist, zum Verstärken der Amplitude des zweiten Signals umfasst, wobei der zweite Verstärker (112) vorzugsweise ein Operationsverstärker ist.

9. Vorrichtung (1) nach einem der Ansprüche 1 bis 6, wobei der vierte Schaltkreis (113) einen digital kontrollierten Verstärker (118) umfasst, der mit dem Ausgang des Instrumentalverstärkers (117) verbunden und dazu konfiguriert ist, eine variable kontrollierte Verstärkung an das Ausgangssignal des Instrumentalverstärkers (117) anzulegen.

10. Vorrichtung (1) nach Anspruch 9, wobei die Steuerung (114) das Ausgangssignal des ersten Verstärkers (111) empfangen kann und den Ausgang des zweiten Verstärkers (112) empfangen kann,
wobei die Steuerung (114) dazu konfiguriert ist, den Widerstand des ersten Verbrauchers (108) mit variablem kontrolliertem Widerstand zu erhöhen, wenn sich die Amplitude des Ausgangs des ersten Verstärkers (111)reduziert, und dazu konfiguriert ist, den Widerstand des ersten Verbrauchers (108) mit variablem kontrolliertem Widerstand zu reduzieren, wenn sich die Amplitude des Ausgangs des ersten Verstärkers (111) erhöht, und
wobei die Steuerung (114) dazu konfiguriert ist, den Widerstand des zweiten Verbrauchers (109) mit variablem kontrolliertem Widerstand zu erhöhen, wenn sich die Amplitude des Ausgangs des zweiten Verstärkers (112)reduziert, und dazu konfiguriert ist, den Widerstand des zweiten Verbrauchers (109) mit variablem kontrolliertem Widerstand zu reduzieren, wenn sich die Amplitude des Ausgangs des zweiten Verstärkers (112) erhöht.

11. Vorrichtung (1) nach Anspruch 1 bis 10, wobei die erste (100), die zweite (101) und/oder die Referenzelektrode (102) trockene Oberflächenelektroden sind, und/oder wobei das erste Signal eine erste Frequenz aufweist, das zweite Signal eine zweite Frequenz aufweist, wobei die erste Frequenz vorzugsweise gleich der zweiten Frequenz ist, und das erste Signal vorzugsweise phasenverschoben zu dem zweiten Signal ist.

12. Verfahren zum Erfassen eines Biopotenzialsignals auf der Haut (500) einer Person, wobei das Verfahren Folgendes umfasst:
Platzieren einer ersten Hautelektrode (100), einer zweiten Hautelektrode (101) und einer Referenzhautelektrode (102) auf der Haut (500) der Person,
Anlegen eines ersten Wechselsignals an die erste Elektrode (100),
Verbinden eines ersten Verbrauchers (108) mit variablem kontrolliertem Widerstand mit der ersten Elektrode (100), Verbinden eines zweiten Verbrauchers (109) mit variablem kontrolliertem Widerstand mit der zweiten Elektrode (101), Messen der Amplitude des ersten Signals und Einstellen des Widerstands des ersten Verbrauchers (108) mit variablem kontrolliertem Widerstand in Abhängigkeit von einer Veränderung der Amplitude des ersten Signals,
Anlegen eines zweiten Wechselsignals an die zweite Elektrode (101),
Messen der Amplitude des zweiten Signals und Einstellen des Widerstands des zweiten Verbrauchers (109) mit variablem kontrolliertem Widerstand in Abhängigkeit von einer Veränderung der Amplitude des zweiten Signals,
Messen des Biopotenzialsignals mit der ersten (100) und der zweiten (101) Elektrode und Anlegen einer variablen kontrollierten Verstärkung an das gemessene Biopotenzialsignal und
Einstellen der variablen kontrollierten Verstärkung (118) in Abhängigkeit von einer Veränderung der Amplitude des gemessenen ersten Signals und der Amplitude des gemessenen zweiten Signals, wenn und nur wenn sich sowohl die Amplitude des ersten Signals als auch die Amplitude des zweiten Signals gleichzeitig verändern.

13. Verfahren nach Anspruch 12, wobei das Festlegen des ersten Verbrauchers (108) mit variablem kontrolliertem Widerstand und des zweiten Verbrauchers (109) mit variablem kontrolliertem Widerstand auf ungefähr die Hälfte des Maximums des vollen Betriebsbereichs des jeweiligen Verbrauchers mit variablem kontrolliertem Widerstand und Messen der Amplitude des ersten Signals und ein Messen der Amplitude des zweiten Signals und Speichern eines ersten anfänglichen Kalibrierungswerts, der dem ersten Wechselsignal zugeordnet ist, und Speichern eines zweiten anfänglichen Kalibrierungswerts, der dem zweiten Wechselsignal zugeordnet ist.

14. Verfahren nach Anspruch 12, wobei das erste Signal eine erste Frequenz aufweist, das zweite Signale eine zweite Frequenz aufweist, wobei die erste Frequenz vorzugsweise gleich der zweiten Frequenz ist, und das erste Signal vorzugsweise phasenverschoben zu dem zweiten Signal ist, und/oder wobei das Biopotenzialsignal, das von der Person produziert wird, ein Elektromyographie(EMG)- oder ein Elektroenzephalographie(EEG)- oder ein Elektrokardiographie(EKG)-Signal ist.

15. Verfahren nach einem der Ansprüche 12 bis 14, umfassend Ausgleichen des folgenden Effekts:
A) Bewegungsartefakte auf der ersten Elektrode (100) durch Einstellen des Widerstands des ersten Verbrauchers (108) mit variablem kontrolliertem Widerstand, Ausgleichen wodurch der Effekt der Bewegungsartefakte auf der zweiten Elektrode (101) durch Einstellen des Widerstands des zweiten Verbrauchers (109) mit variablem kontrolliertem Widerstand ausgeglichen wird, und/oder
B) Schweißansammlung auf der Haut der Person durch Erhöhen der variablen kontrollierten Verstärkung (118).

## Revendications

1. Appareil (1) pour détecter un signal de biopotentiel sur la peau (500) d'une personne utilisant au moins une première électrode cutanée (100), une deuxième électrode cutanée (101) et une électrode cutanée de référence (102) pour un placement espacé sur ladite peau (500) de ladite personne, l'appareil (1) comprenant :
- une première borne (103) pour une liaison à ladite première électrode (100),
- une deuxième borne (104) pour une liaison à ladite deuxième électrode (101),
- un premier circuit (110) configuré pour mesurer le signal de biopotentiel provenant de ladite première borne (103) et de ladite deuxième borne (104),
- une troisième borne (105) permettant une liaison à ladite électrode cutanée de référence (102),
- une première charge de résistance variable commandée (108) reliée à ladite première borne (103),
- une deuxième charge de résistance variable commandée (109) reliée à ladite deuxième borne (104),
- un premier générateur de signal (106) permettant de générer un premier signal alternatif relié à ladite première borne (103),
- un deuxième générateur de signal (107) permettant de générer un deuxième signal alternatif relié à ladite deuxième borne (104),
- un quatrième circuit (113) relié à la sortie dudit premier circuit (110) et configuré pour appliquer un gain variable commandé (118) au signal de sortie dudit premier circuit (110),
- un dispositif de commande (114) recevant :
• un signal de sortie dudit premier circuit (110),
• un signal comprenant une composante représentative de l'amplitude dudit premier signal alternatif,
• un signal comprenant une composante représentative de l'amplitude dudit deuxième signal alternatif,
- le dispositif de commande (114) étant configuré :
• pour déterminer l'amplitude dudit premier signal alternatif,
• pour déterminer l'amplitude dudit deuxième signal alternatif,
• pour commander ladite première charge de résistance variable commandée (108) et ladite deuxième charge de résistance variable commandée (109),
• pour commander ledit gain variable commandé (118),
• pour adapter la charge de ladite première charge de résistance variable commandée (108) en fonction d'un changement de l'amplitude déterminée dudit premier signal alternatif,
• pour adapter la charge de ladite deuxième charge de résistance variable commandée (109) en fonction d'un changement de l'amplitude déterminée dudit deuxième signal alternatif, et
• pour ajuster ledit gain variable commandé (118) en fonction de l'amplitude déterminée dudit premier signal alternatif et de l'amplitude déterminée dudit deuxième signal alternatif, lorsque, et seulement lorsque, l'amplitude déterminée desdits premier signal alternatif et deuxième signal alternatif changent simultanément.

2. Appareil selon la revendication 1, dans lequel le signal comprenant au moins une composante représentative de l'amplitude dudit premier signal alternatif et le signal comprenant une composante représentative de l'amplitude dudit deuxième signal alternatif est le signal de sortie dudit premier circuit (110).

3. Appareil selon la revendication 1 ou 2, dans lequel le dispositif de commande est configuré :
- pour mettre en œuvre une analyse temps-fréquence du signal de sortie dudit premier circuit (110) et déterminer ainsi la partie du signal de sortie dudit premier circuit (110) provenant dudit premier signal alternatif et la partie du signal de sortie dudit premier circuit (110) provenant dudit deuxième signal alternatif,
- pour déterminer l'amplitude du signal de sortie dudit premier circuit (110) provenant dudit premier signal alternatif,
- pour déterminer l'amplitude du signal de sortie dudit premier circuit (110) provenant dudit deuxième signal alternatif,
- pour commander ladite première charge de résistance variable commandée (108),
- pour commander ladite deuxième charge de résistance variable commandée (109),
- pour commander ledit gain variable commandé (118),
- pour adapter la charge de ladite première charge de résistance variable commandée (108) en fonction de l'amplitude du signal de sortie dudit premier circuit (110) provenant dudit premier signal alternatif,
- pour adapter la charge de ladite deuxième charge de résistance variable commandée (109) en fonction de l'amplitude du signal de sortie dudit premier circuit (110) provenant dudit deuxième signal alternatif, et
- ajuster ledit gain variable commandé (118) en fonction de l'amplitude du signal de sortie dudit premier circuit (110) provenant dudit premier signal alternatif et de l'amplitude du signal de sortie dudit premier circuit (110) provenant dudit deuxième signal alternatif lorsque, et seulement lorsque, l'amplitude de signal de sortie dudit premier circuit (110) provenant dudit premier signal alternatif et l'amplitude du signal de sortie dudit premier circuit (110) provenant dudit deuxième signal alternatif changent simultanément.

4. Appareil (1) selon la revendication 1 à 3, comprenant un deuxième circuit (115) relié à ladite première borne (103) pour détecter et/ou amplifier l'amplitude dudit premier signal et un troisième circuit (116) relié à ladite deuxième borne (104) pour détecter et/ou amplifier l'amplitude dudit deuxième signal, un dispositif de commande (114) recevant le signal de sortie dudit deuxième circuit (115) et recevant le signal de sortie dudit troisième circuit (116), ledit dispositif de commande (114) étant configuré :
- pour adapter la charge de ladite première charge de résistance variable commandée (108) en fonction de l'amplitude dudit signal de sortie dudit deuxième circuit (115),
- pour adapter la charge de ladite deuxième charge de résistance variable commandée (109) en fonction de l'amplitude dudit signal de sortie dudit troisième circuit (116), et
- pour ajuster ledit gain variable commandé (118) en fonction de l'amplitude dudit signal de sortie dudit deuxième circuit (115) et de l'amplitude du signal de sortie du troisième circuit (116) lorsque, et seulement lorsque, l'amplitude des signaux de sortie desdits deuxième circuit (115) et troisième circuit (116) changent simultanément.

5. Appareil (1) selon la revendication 1 à 4, dans lequel ledit dispositif de commande (114) est configuré pour :
A) augmenter ledit gain variable commandé (118) lorsque et seulement lorsque l'amplitude des signaux de sortie desdits deuxième (115) et troisième (116) circuits s'atténue simultanément, et/ou
B) diminuer ledit gain variable commandé (118) lorsque et seulement lorsque l'amplitude des signaux de sortie des deuxième (115) et troisième (116) circuits augmentent simultanément, et/ou
C) régler la première charge de résistance variable commandée (108) et la deuxième charge de résistance variable commandée (109) toutes deux à une valeur moyenne, et en ce que le dispositif de commande (114) est configuré pour ensuite étalonner l'appareil en fonction de l'amplitude du signal de sortie du deuxième circuit (115) et en fonction du signal de sortie du troisième circuit (116), et dans lequel le dispositif de commande (114) est de préférence configuré pour stocker une première valeur d'étalonnage initiale associée au premier signal alternatif et est de préférence configuré pour stocker une deuxième valeur d'étalonnage initiale associée au deuxième signal alternatif.

6. Appareil (1) selon la revendication 5 option C), dans lequel le dispositif de commande (114) est configuré pour mettre à jour la première et la deuxième valeur d'étalonnage initiale après que le dispositif de commande a ajusté le gain variable commandé (118).

7. Appareil (1) selon l'une quelconque des revendications 1 à 3, dans lequel le premier circuit (110) comprend un amplificateur instrumental (117) relié à la première borne (103) et à la deuxième borne (104) pour amplifier le signal de biopotentiel.

8. Appareil (1) selon l'une quelconque des revendications 1 à 5, dans lequel le deuxième circuit (115) comprend un premier amplificateur (111) relié à la première borne (103) pour amplifier l'amplitude du premier signal, le premier amplificateur (111) étant de préférence un amplificateur opérationnel, ou dans lequel le troisième circuit (116) comprend un deuxième amplificateur (112) relié à la deuxième borne (104) pour amplifier l'amplitude du deuxième signal, le deuxième amplificateur (112) étant de préférence un amplificateur fonctionnel.

9. Appareil (1) selon l'une quelconque des revendications 1 à 6, dans lequel le quatrième circuit (113) comprend un amplificateur à commande numérique (118) relié à la sortie de l'amplificateur instrumental (117) et configuré pour appliquer un gain variable commandé au signal de sortie de l'amplificateur instrumental (117).

10. Appareil (1) selon la revendication 9, dans lequel le dispositif de commande (114) reçoit le signal de sortie du premier amplificateur (111) et reçoit la sortie du deuxième amplificateur (112),
le dispositif de commande (114) étant configuré pour augmenter la résistance de la première charge de résistance variable commandée (108) lorsque l'amplitude de la sortie du premier amplificateur (111) diminue et configuré pour diminuer la résistance de la première charge de résistance variable commandée (108) lorsque l'amplitude de la sortie du premier amplificateur (111) augmente, et
le dispositif de commande (114) étant configuré pour augmenter la résistance de la deuxième charge de résistance variable commandée (109) lorsque l'amplitude de la sortie du deuxième amplificateur (112) diminue et configuré pour diminuer la résistance de la deuxième charge de résistance variable commandée (109) lorsque l'amplitude de la sortie du deuxième amplificateur (112) augmente.

11. Appareil (1) selon la revendication 1 à 10, dans lequel la première électrode (100), la deuxième électrode (101) et/ou l'électrode de référence (102) sont des électrodes de surface sèches et/ou, dans lequel le premier signal a une première fréquence, le deuxième signal a une deuxième fréquence, la première fréquence étant de préférence égale à la deuxième fréquence, et le premier signal étant de préférence déphasé par rapport au deuxième signal.

12. Procédé de détection d'un signal de biopotentiel sur la peau (500) d'une personne, le procédé comprenant :
le placement d'une première électrode cutanée (100), d'une deuxième électrode cutanée (101) et d'une électrode cutanée de référence (102) sur la peau (500) de la personne,
l'application d'un premier signal alternatif à la première électrode (100),
la liaison d'une première charge de résistance variable commandée (108) à la première électrode (100),
la liaison d'une deuxième charge de résistance variable commandée (109) à la deuxième électrode (101),
la mesure de l'amplitude du premier signal et l'ajustement de la résistance de la première charge de résistance variable commandée (108), en fonction d'un changement d'amplitude du premier signal,
l'application d'un deuxième signal alternatif à la deuxième électrode (101),
la mesure de l'amplitude du deuxième signal et l'ajustement de la résistance de la deuxième charge de résistance variable commandée (109) en fonction d'un changement d'amplitude du deuxième signal,
la mesure du signal de biopotentiel avec la première électrode (100) et la deuxième électrode (101) et l'application d'un gain variable commandé au signal de biopotentiel mesuré, et
l'ajustement du gain variable commandé (118) en fonction d'un changement d'amplitude du premier signal mesuré et de l'amplitude du deuxième signal mesuré lorsque et seulement lorsque l'amplitude du premier signal et l'amplitude du deuxième signal changent simultanément.

13. Procédé selon la revendication 12, dans lequel le réglage de la première charge de résistance variable commandée (108) et de la deuxième charge de résistance variable commandée (109) à environ la moitié du maximum de la plage entièrement fonctionnelle de la charge de résistance variable commandée respective et la mesure de l'amplitude du premier signal et une mesure de l'amplitude du deuxième signal, et le stockage d'une première valeur d'étalonnage initiale associée au premier signal alternatif et le stockage d'une deuxième valeur d'étalonnage initiale associée au deuxième signal alternatif.

14. Procédé selon la revendication 12, dans lequel le premier signal a une première fréquence, le deuxième signal a une deuxième fréquence, la première fréquence étant de préférence égale à la deuxième fréquence, et le premier signal étant de préférence déphasé par rapport au deuxième signal, et/ou
dans lequel le signal de biopotentiel produit par la personne est un signal d'électromyographie (EMG) ou d'électroencéphalographie (EEG) ou d'électrocardiographie (ECG).

15. Procédé selon l'une quelconque des revendications 12 à 14, comprenant la compensation de l'effet de :
A) des artefacts de mouvement sur la première électrode (100) en ajustant la résistance de la première charge de résistance variable commandée (108), la compensation de l'effet des artefacts de mouvement sur la deuxième électrode (101) en ajustant la résistance de la deuxième charge de résistance variable commandée (109) et/ou
B) l'accumulation de sueur sur la peau de la personne, en augmentant le gain variable commandé (118).
